# FASCICULE DE BREVET EUROPEEN

(11) **EP 2 279 764 B1**
(45) Date de publication et mention de la délivrance du brevet: **27.06.2012**
(21) Numéro de dépôt: 10003557.5
(22) Date de dépôt: 26.10.2006
(51) Int. Cl.: A61L 27/06, A61L 27/56, A61L 31/02, A61F 2/30, A61F 2/04, A61K 6/00, A61C 8/00, A61C 13/20, A61F 2/28, A61K 6/04

(54) **Procédé d'obtention d'un implant composite biocompatible.**
Verfahren zum Erhalt eines biokompatiblen Komposit-implantats
Method for obtaining a biocompatible composite implant

(30) Priorité: 27.10.2005 FR 0511005
(43) Date de publication de la demande: 02.02.2011
(62) Demande divisionnaire de: 06831024.2
(73) Titulaire: PROTIP SAS, 67380 Lingolsheim (FR); UNIVERSITE LOUIS PASTEUR, 67000 Strasbourg (FR); Hôpitaux Universitaires de Strasbourg, 67000 Strasbourg (FR)
(72) Inventeur: Walder, André, 94240 L'Hay les Roses (FR); Debry, Christian, 75015 Paris (FR)
(74) Mandataire: Barny, Luc

(56) Documents cités:
- EP-A- 0 856 299
- WO-A-02/066693
- US-A- 4 374 669
- US-A- 4 550 448
- US-A- 5 855 612
- US-A1- 2005 129 949
- PATENT ABSTRACTS OF JAPAN vol. 014, no. 43 (C-681), 26 janvier 1990 (1990-01-26) & JP 01, 275766, A, (SEIKO EPSON CORP), 6 novembre 1989 (1989-11-06)

## Description

La présente invention se rapporte au domaine des prothèses métalliques implantables chez l'homme ou chez l'animal.

La présente invention concerne plus spécifiquement un procédé de fabrication d'un implant métallique implantable chez l'homme ou chez l'animal dont la porosité ouverte permet la colonisation par des cellules vivantes, jouant ainsi le rôle d'une structure support permettant la reconstruction naturelle de l'organe disparu à la suite d'une opération ou d'un choc traumatique.

Il peut en particulier s'agir de prothèses laryngées totales destinées à permettre la reconstruction d'un larynx chez les malades ayant subi une laryngectomie, i.e une ablation totale du larynx par exemple à la suite d'un cancer.

De manière plus générale, le domaine de la présente invention s'étend à toutes les prothèses de reconstruction locale concernant une paroi osseuse telle que la calotte crânienne, les mandibules, ou encore la paroi thoracique.

Encore plus spécifiquement, la présente invention concerne un procédé de fabrication d'un implant composite associant des microsphères de titane ou d'alliage à base de titane ou d'alliage biocompatible avec des pièces de titane massif ou d'alliage à base de titane ou d'alliage biocompatible.

Il existe, à ce jour, diverses techniques mettant en oeuvre le titane ou les alliages à base de titane pour obtenir des prothèses biocompatibles i.e tolérées et non dégradées par l'organisme vivant dans lequel elles sont implantées.

Le titane et les alliages à base de titane présentent en effet l'avantage d'être légers, résistants à la corrosion, donc résistants aux fluides circulant dans les organismes vivants et présentent en outre de bonnes propriétés mécaniques.

Ils permettent donc d'envisager des implants solides, même s'ils sont de taille conséquente, peu allergisants et peu susceptibles de créer des réactions inflammatoires.

Tout ceci est connu de l'art antérieur.

On peut citer, à titre d'exemple, l'utilisation du titane massif pour faire des prothèses de hanche ou de genoux.

Un tel dispositif, présenté cependant les inconvénients inhérents au titane massif à savoir que la structure de sa surface ne permet pas l'adhésion ni la colonisation par les tissus adjacents à la prothèse. De sorte qu'une fois implantée dans un corps vivant la prothèse aura tendance à se désolidariser de son support vivant, osseux ou autre, après une période plus ou moins longue.

Plus récemment, ont été développées des prothèses à base de matériaux inorganiques tels que l'hydroxyapatite ou des verres dits bioactivés dans la mesure où ils ont montré certaines capacités à être colonisés par les tissus vivants, notamment les tissus osseux.

Cependant ces prothèses ne présentaient pas les qualités mécaniques comparables à celles réalisées en titane massif ou en alliage à base de titane massif.

L'idéal est que le chirurgien puisse disposer d'une prothèse présentant les qualités mécaniques du titane massif ou de l'alliage à base de titane, d'une part et de bioactivité, d'autre part.

Il a donc été imaginé, dans l'art antérieur, de réaliser des implants constitués d'une âme en titane massif revêtue d'une couche d'hydroxyapatite ou de verre bioactif.

En sus de sa complexité de réalisation ce dispositif présentait en outre des défauts dans l'adhérence de la couche de surface de l'âme du dispositif.

Il a cependant été remédié à ce nouvel inconvénient par des procédés plus ou moins complexes. A titre d'exemples non limitatifs, on peut citer l'invention décrite dans la demande de brevet JP N° 1-275766. Ce procédé de production complexe permet notamment, après deux étapes en solution et une étape de chauffage, d'améliorer l'adhérence de la couche bioactive sur l'âme en titane massif.

On peut également citer la méthode du traitement de surface du titane massif ou de l'alliage à base de titane décrite par B.Walivaara, I. Lundstrom and P. Tengvall dans Clinical Materials (Vol. 12, pages 141-148). Cette dernière méthode présente cependant le désavantage de n'aboutir qu'à un revêtement extrêmement fin de l'âme de l'implant tout à fait insuffisant pour permettre une colonisation effective des tissus.

On peut également citer la demande de brevet US 5 855 612 qui décrit un procédé dont l'objectif est d'obtenir un film d'apatite à la surface de l'âme de la prothèse. Pour se faire, l'invention propose de traiter l'âme de la prothèse par immersion dans une solution aqueuse de peroxyde d'hydrogène.

Il n'en reste pas moins vrai que les inconvénients mentionnés plus haut persistent et notamment qu'il ne peut être obtenu une colonisation tissulaire rapide et pérenne en milieu septique.

Ce défaut, rédhibitoire pour le chirurgien, s'explique notamment par le fait que la taille des pores obtenus n'étant contrôlable pour aucun des procédés précités se sont les microorganismes, le plus souvent pathogène, de taille appropriés qui sont favorisés pour s'y loger en premier.

Les microorganismes pathogènes étant de taille extrêmement variée, et les tissus susceptibles de coloniser la surface de la prothèse étant d'une taille unique, il est aisé de comprendre que la compétition dans la colonisation de la surface n'est que très rarement gagnée par ces derniers.

Il s'en suit des infections, des complications chirurgicales sévères et dans certains cas le décès du patient.

Un autre perfectionnement aux implants en titane massif ou alliages à base de titane massif est proposé dans la demande de brevet EP 0 856 299 qui décrit le recours à l'utilisation de microsphères de titane, d'alliages à base de titane ou de matériaux biocompatibles, solidarisées entre elles pour aboutir à une prothèse dont les caractéristiques dimensionnelles et l'état de surface sont déterminant pour assurer après implantation de ladite prothèse *in vivo* dans un environnement non stérile, une colonisation cellulaire ou tissulaire plus rapide qu'une éventuelle colonisation par un microorganisme pathogène.

La porosité est effectivement présente dans l'épaisseur même de la structure de l'implant et la taille des pores peut être prédéterminée par le choix du calibre des micro-billes utilisées.

Selon l'invention décrite dans la demande de brevet EP 0 856 299 une telle prothèse métallique à porosité ouverte peut être soumise à colonisation complète. Il s'agit alors d'une structure rigide susceptible de constituer un véritable support de l'organe à reconstituer acceptant une implantation dans un environnement non aseptique en raison de leur porosité ouverte qui permet une colonisation tissulaire rapide, les cellules vivantes créant entre elles un véritable réseau de maillage englobant les microsphères.

Si cette demande de brevet apporte effectivement un avantage indéniable, elle ne règle cependant que partiellement le problème, à savoir que ces implants ont perdu une partie des qualités mécaniques, notamment la souplesse du titane massif ou des alliages à bases de titane massif et/ou autres métaux biocompatibles.

Ainsi les prothèses réalisées en titane microporeux et soumises à des forces importantes risquent de casser. Il est notamment fait référence ici aux implants dentaires et aux mandibules.

Un autre inconvénient de ces prothèses obtenues par traitement thermiques de microbilles est que la porosité inhérente à sa structure rend impossible les opérations d'usinage classique telles que le filetage et le taraudage interdisant ainsi de nombreux modes de réalisation.

Il est notamment extrêmement difficile d'imaginer des modes de réalisation permettant d'intégrer des brides ou des points d'encrage permettant de fixer la prothèse à son environnement *in vivo.*

Cette limitation est en outre aggravée par le fait que les qualités de résistance mécanique du matériau interdissent, si l'on perce des trous dans la prothèse, de les soumettre à des forces significatives et notamment de les utiliser pour fixer des éléments liant la prothèse à son environnement.

L'art antérieur ne présente donc à ce jour aucune solution remédiant à l'ensemble des inconvénients décrits plus haut.

Enfin le procédé d'obtention décrit dans la demande EP 0 856 299 comporte une étape de « frittage par électro-étincelage » faisant appel à un effet Joule important nécessitant une quantité d'énergie surfacique importante à savoir 20 à 80 J/mm2.

L'inconvénient majeur d'une méthode utilisant un effet Joule important est que la fusion de métal obtenue au point de contact des billes peut être excessive. Dans ce cas des fissures liées à un retrait dimensionnel excessif de la pièce, peuvent se former perpendiculairement au sens du courant.

Ceci est très net dans le cas de plaques minces et de tubes pour lesquels le rapport surface/volume est grand. La pièce fissurée est bien entendu inutilisable. La fusion excessive du métal, liée à cette méthode, peut aller jusqu'à la fusion complète de plusieurs microsphères avec formation de gouttes de métal fondu. Ces gouttes de métal fondu induisent une perte de porosité qui rend là aussi l'implant impropre à l'usage auquel il est destiné. Cet inconvénient est d'autant plus marqué que la taille des billes est réduite.

Par ailleurs un tel procédé faisant appel à des quantités d'énergie très importantes conduit à la formation d'un col de soudure, au point de contact entre les billes, lui même de taille importante. Un surdimensionnement du col de soudure peut s'opposer à ce qu'il soit procédé par la suite à une opération de frittage classique.

En effet une telle opération de frittage qui abouti au renforcement de la soudure par migration d'atomes de la surface d'une particule à une autre nécessite de porter la pièce à une température qui est proportionnelle à l'épaisseur de la matière considérée.

En pratique, un col de soudure trop épais peut interdire toute opération de frittage ultérieure dans la mesure où elle supposerait que la pièce soit portée à une température supérieure au point de fusion du métal considéré ou du moins qu'un tel point de fusion soit dépassé pour telle ou telle partie de la pièce. Il s'ensuit là aussi une perte de porosité.

Le procédé décrit dans la demande EP 0 856 299 pouvant rendre difficile une étape de consolidation par traitement thermique ultérieur, la solidité des implants confectionnés par sa mise en oeuvre doit être d'emblée acquise, ce qui rend sa mise au point complexe. En outre le procédé décrit dans la demande EP 0 856 299 utilisant des énergies élevées les électrodes se retrouvent très fortement soudées à la pièce.

Il en d'écoule une certaine complexité de l'opération de démoulage avec des risques de détérioration de la pièce moulée et une détérioration certaine des électrodes. Cette soudure des électrodes à l'implant impose en tout état de cause que les parties soudées de l'implant soient, par la suite, usinées de façon adéquate pour présenter une surface sans défaut apparent. Un tel usinage, délicat car opéré sur une pièce fragile, augmente le coût de celle-ci.

Par ailleurs lorsque la soudure de l'électrode de l'implant est trop prononcée il en résulte une perte de porosité qui rend cette dernière impropre à l'usage auquel elle est destinée.

En conclusion la méthode décrite dans la demande de brevet EP 0 856 299 présente de plusieurs inconvénients qui la rendent difficilement applicable à un certaines formes d'implants, en outre même lorsqu'elle est applicable la résistance mécanique des pièces obtenues reste relativement faible.

La présente invention se propose de pallier à l'ensemble des inconvénients décrits et propose un implant biocompatible composite totalement innovant et reposant sur un concept inventif neuf et, jusqu'à aujourd'hui, jamais décrit ni même suggéré dans l'art antérieur.

En effet, l'objectif premier de la demanderesse est de fournir un implant métallique composite obtenu par un procédé de réalisation d'implants ayant dés qualités mécaniques irréprochables tout en offrant une biocompatibilité complète permettant ainsi de limiter considérablement les risques de bris et les risques d'infection.

Pour ce faire, la demanderesse a développé un procédé allant à l'encontre de l'ensemble des préjugés et des recherches actuelles qui tendent, comme décrit plus haut dans l'art antérieur, soit à revêtir le titane massif d'une fine couche biocompatible, soit à réaliser des implants qui sont poreux dans leur masse et obtenus par électro-étincelage à forte énergie.

La présente invention explore une toute autre alternative puisqu'elle envisage, pour la première fois, l'utilisation d'un implant constitué d'au moins une âme en métal massif tel que par exemple le titane, un alliage à base de titane ou un alliage biocompatible et d'une superstructure métallique à porosité ouverte obtenue par solidarisation de microsphères.

La présente invention se situe dans une voie diamétralement opposée aux investigations existantes puisqu'il est choisi de noyer de fins éléments de métal massif dans un important volume de métal à porosité ouverte, là où l'état de la technique proposait de recouvrir le métal massif d'un fin revêtement bioactif.

Selon une première forme de réalisation la présente invention consiste en un implant métallique composite obtenu par un procédé d'obtention d'un implant métallique de soutien et/ou de remplacement tissulaire à porosité ouverte, caractérisé en ce qù'il comprend les étapes suivantes :
- (i) sélectionneur un moule (1), non conducteur, de forme appropriée correspondant à l'implant souhaité,
- (ii) disposer dans ledit moule au moins une âme massive métallique(7),
- (iii) remplir le volume du moule (1) restant disponible par de la poudre de microsphères (3), et
- (iv) solidariser les microsphères (3) entre elles ainsi qu'à ladite au moins une âme massive (7) par électro-étincelage,
ladite étape iv) d'électro-étincelage étant réalisée par décharge d'un courant électrique, d'une tension prédéterminée de manière à obtenir une densité surfacique d'énergie J prédéterminée entre, d'une part, au moins une première électrode (5) en périphérie du moule (1) et, d'autre part, au moins une seconde électrode (5') de polarité opposée et positionnée de manière à ne pas former court-circuit avec ladite au moins une âme massive métallique (7).

Par « âme massive », il faut comprendre une pièce, tel un insert, substantiellement massif dans sa structure c'est-à-dire non constitué d'un agencement d'éléments telles des microsphères ou des fibres de granulométrie ou de section inférieure au millimètre et constitué en métal tel que le titane, l'alliage à base de titane ou tout alliage biocompatible. Une âme massive peut cependant être constituée d'un agencement de fils de titane de section sensiblement égale ou supérieure au millimètre.

Par « microsphères » ou « poudre de microsphères » il faut comprendre une poudre obtenue par pulvérisation à l'électrode tournante (« Powder Metallurgy of Superalloys », G.H GESSINGER, Butterworths Mnographs in Materials, pages 29 à 32), ou tout autre procédé équivalent donnant des sphères avec peu ou pas d'aspérités, d'une poudre constituée par des microsphères métalliques de titane ou d'alliages à base de titane ou d'alliages biocompatibles présentant une géométrie parfaitement sphérique, et/ou un état de surface pratiquement dépourvu d'irrégularité telles que décrites dans la demande EP 0 856 299 A1. Un tel procédé consiste à fondre, soit à l'aide d'un arc électrique ou d'une torche à plasma sous gaz neutre, soit par un faisceau d'électrons sous vide, la surface frontale d'un cylindre en rotation rapide autour de son axe. Le liquide ainsi formé migre, sous l'action des forces d'inertie centrifuges, vers la périphérie du lingot en rotation, d'où il s'échappe sous forme de gouttelettes qui se refroidissent et se solidifient pendant leur vol dans le gaz neutre de l'enceinte.

Par « électrode », il faut entendre l'extrémité d'un conducteur, préférentiellement en laiton relié d'une part, à l'un des pôles du système de condensateur générant la décharge électrique et d'autre part , en contact avec une partie métallique de l'implant à former telle qu'un ensemble de microsphères en contact les unes avec les autres et/ou une âme massive. Une « électrode » s'entend comme une électrode de polarité donnée ou une série d'électrodes de même polarité.

Par « moule de forme appropriée » il faut entendre un moule reproduisant exactement la forme de la pièce à mouler.

Par « électro-étincelage » il faut comprendre toute opération visant à souder les microsphères et au moins une âme massive par décharge d'un courant électrique au travers de la pièce à souder. La publication « Preforming using high-voltage electrical discharge », S.T.S Al-Hassani& T.J.Davies, Powder Metallurgy, 1980 décrit un procédé d'électro-étincelage.
Dans le cas d'une réalisation en titane pour fabriquer une pièce de forme donnée, la poudre de titane, qui a été tamisée pour obtenir la fraction granulométrique souhaitée, est versée dans un moule en polymère non conducteur, reproduisant exactement la forme de la pièce à produire, des électrodes ayant été placées préalablement à des endroits appropriés, préférentiellement mais pas nécessairement, en vis-à-vis ou quasi vis-à-vis de part et d'autre du moule, au contact de la poudre ou d'un élément en titane massif dans le cas de la mise en place d'une âme située en extrémité de pièce. Ces électrodes sont reliées aux polarités d'un banc de condensateurs chargés sous haute tension.
Par action sur un interrupteur, le courant de décharge des condensateurs traverse le volume de poudre. Il est obligé de passer par les points de contact entre particules, c'est à dire par une section infiniment petite. Il se produit localement, à ces points de contact, un échauffement par effet Joule (V volts = R ohms x I ampères et P watts = R ohms x I2 ampères) qui, si l'intensité du courant de décharge est suffisante, peut conduire à la fusion locale du métal ou de l'alliage.

Selon ce procédé les prothèses métalliques présentent, à l'exception des parties réalisées en métal massif, une superstructure à porosité ouverte qui se caractérise par des espaces intersphéroïdaux présentant une dimension sensiblement égale au tiers du diamètre des poudres.

Cette porosité est tout à fait propice à la colonisation tissulaire et il est particulièrement surprenant de constater que même les interfaces entre les parties de la prothèse constituées de métal à porosité ouverte et les parties constituées de métal massif présentent une solidarisation par soudure homogène. Il était en effet à craindre que, le métal massif étant bien meilleur conducteur que les microsphères de ce même métal, des défauts de soudure soient constatés suite à l'étape d'électro-étincelage.

Ainsi il pouvait être anticipé que lorsque le courant choisit préférentiellement le métal massif pour son passage, les parties de la superstructure de microsphères situées latéralement à ces lignes de courant ne soient mal soudées ou pas soudées du tout du fait d'un effet Joule insuffisant.

A l'inverse il pouvait également être anticipé des effets de fonte des microsphères dus à un effet Joule trop important pour les parties de superstructure composées des microsphères métalliques situées dans l'axe des lignes de courant.

L'invention va au delà de ces préjugés et montre qu'il est possible d'obtenir un soudage homogène par électro-étincelage avec une disposition favorable des âmes métalliques dès lors qu'une quantité d'énergie appropriée est utilisée. Cette quantité d'énergie se situe préférentiellement entre 1 et 10 J/mm2 et plus préférentiellement entre 3 et 8 J/mm2.

L'invention apporte une autre solution particulièrement favorable en proposant d'utiliser une extrémité d'au moins une des âmes métalliques comme électrode pour le procédé d'électro-étincelage.

Cette solution permet d'assurer une diffusion particulièrement optimum de la décharge électrique dans la pièce à souder. Elle évite en outre l'utilisation d'au moins une électrode classique en contact avec les microsphères et épargne ainsi les inconvénients usuellement liés à cet emploi tels qu'ils sont décrits ci-dessus.

En effet l'utilisation d'une électrode classique engendre des coûts importants liés d'une part à l'entretient et au remplacement de l'électrode qui est fonction de l'intensité de la décharge et d'autre part aux opérations d'usinage telles que le meulage et le polissage des surfaces de l'implant, fondues où bleuies dans leur portion située au regard de l'électrode.

Même lorsqu'une âme massive ne sert pas directement d'électrode dans le sens où elle n'est pas directement reliée à la source d'énergie elle peut servir d'électrode intermédiaire au sein même de la pièce à souder. En effet ces âmes massives constituent des zones de conductibilité électrique plus élevées que les zones constituées de poudre de microsphères. Ces âmes massives, judicieusement disposées peuvent donc servir à diffuser de façon plus homogène le courant électrique dans la pièce à souder. Ainsi sans augmenter la quantité d'énergie un soudage de qualité peut être obtenu de façon homogène. Par rapport à l'art antérieur les quantités d'énergie utilisées sont divisées quasiment par dix.

Par disposition « judicieuse » on entend toute disposition des âmes massives qui permet une diffusion homogène et optimale du courant afin que celui-ci atteigne, dans l'ensemble de la pièce la valeur critique à partir de laquelle il y a fusion aux points de contact. Une disposition judicieuse d'une âme massive donnée exclu une disposition où celle-ci fait court-circuit ou quasi court circuit entre deux électrodes de polarités opposées. Au contraire une disposition judicieuse d'une âme massive sera obtenue lorsque l'interface entre celle-ci est la poudre de microsphère sera substantiellement perpendiculaire aux lignes de circulation du courant entre les électrodes de polarités opposées.

La quantité d'énergie stockée dans les condensateurs est donnée par la relation : E joules = ½ C farads x V2 volts.

L'intensité du courant de décharge des condensateurs est proportionnelle à l'énergie emmagasinée, ce qui signifie qu'avec un banc de condensateurs de capacité donnée, il est possible de faire varier la quantité d'énergie, donc l'intensité du courant de décharge, en agissant sur la tension de charge, qui par ailleurs intervient au carré, donc conduit à un accroissement important de la quantité d'énergie stockée pour une augmentation de la tension.

L'énergie nécessaire pour souder les billes entre elles dépend de la section de passage du courant au travers de la pièce, donc de la taille de celle-ci. Elle dépend aussi de la résistance ohmique du matériau, de son point de fusion et de la taille des particules de poudre. En effet, à section de passage équivalente, plus les particules sont fines, plus le nombre de point de contact sont nombreux et plus le courant de décharge est divisé entre de nombreux circuits. Il est alors nécessaire d'augmenter le courant de décharge par un accroissement de l'énergie stockée, pour obtenir le même résultat.

Classiquement l'énergie nécessaire peut varier d'environ 40 joules pour une petite pièce à plusieurs centaines, voire plusieurs milliers de joules pour une grosse pièce. Dans la pratique il est nécessaire de disposer les électrodes de façon à faire passer le courant par une section la plus petite possible, pour réduire l'énergie nécessaire et, d'un point de vue industriel, la taille de la machine de soudure.

Dans la réalité l'énergie récupérée ne correspond cependant pas exactement au calcul théorique donnant la charge des condensateurs, car il peut se produire des pertes dans le circuit de décharge par différents phénomènes comme des effets de self-induction ou des résistances parasites. L'adaptation de la quantité d'énergie nécessaire pour obtenir une consolidation du matériau est obtenue empiriquement en vérifiant la cohésion des billes de titane par des essais mécaniques d'écrasement. La durée de la décharge est très faible, 5 à 10 µsec (millionièmes de seconde), mais l'intensité du courant qui provoque la fusion locale et la soudure des billes est très élevée, plusieurs milliers à plusieurs dizaines de milliers d'ampères selon la taille de la pièce. La détermination exacte des paramètres électriques est indispensable pour satisfaire les besoins de reproductibilité des pièces.

La température atteinte aux points de contact entre les billes est très élevée ; elle dépasse le point de fusion du titane (1 660 °C) et provoque même la vaporisation d'une très faible quantité de métal, mais la brièveté de la décharge, la petite taille du volume fondu et la diffusion de la chaleur produite dans le volume des billes, font que la température moyenne de la pièce reste très basse, entre 40 à 60 °C.

L'invention propose ainsi comme mode de réalisation que ladite première électrode (5) et ladite au moins seconde électrode (5') soient positionnées par rapport à l'âme métallique (7) de manière à ce quelles lignes de courant formées entre ces deux électrodes ne soient pas parallèles à l'interface microsphères/âme métalliques (7) de plus grande surface.

L'invention propose également comme mode de réalisation que ladite première électrode (5) et ladite au moins seconde électrode (5') sont positionnées par rapport à l'âme métallique (7) de manière à ce que les lignes de courant formées entre ces deux électrodes soient sensiblement perpendiculaires a l'interface microsphères/âme métalliques (7) de plus grande surface.

L'invention propose également comme mode de réalisation qu'une électrode, d'une polarité donnée soit disposée de manière à venir en contact avec au moins une âme massive (7) et l'autre électrode de polarité opposée soit disposée de manière à venir en contact avec les microsphères.

L'invention propose également comme mode de réalisation qu'une électrode, de polarité donnée, soit disposée de manière à venir en contact avec au moins une âme massive (7) et l'autre électrode, de polarité opposée est disposée de manière à venir en contact avec au moins une autre âme massive (7').

La diffusion optimale du courant dans l'implant permet, suite à l'opération d'électro-étincelage, d'obtenir entre les microsphères, d'une part et les microsphères et la surface des âmes, d'autre part, des cols de soudure de taille particulièrement homogène. Cette maîtrise de la taille des cols de soudure permet en outre d'envisager de faire suivre l'opération d'électro-étincelage d'une opération de frittage en phase solide sans que l'énergie nécessaire au chauffage des pièces et proportionnelle à la taille des cols ne fasse craindre que le point de fusion du métal utilisé ne soit atteint.

Cette opération de frittage en phase solide permet de consolider de manière importante les implants proposés et constitue un avantage indéniable.

Ainsi dans un mode préféré de réalisation de l'invention le procédé est complété par une étape de frittage en phase solide où la pièce est portée, dans un four à vide poussé, à une température de 1200°C à 1400°C, préférentiellement 1250°C pendant une durée de 3h à 15h, préférentiellement 12h. L'opération de frittage consiste à chauffer l'implant à une température telle que les grains de poudre se soudent entre eux par des déplacements d'atome à l'état solide.

Il est important de noter que les microsphères étant solidarisées entre-elles et à l'âme métallique suite à l'opération d'électro-étincelage, l'implant peut être fritté après démoulage. Ceci constitue un avantage considérable par rapport aux procédés existants, notamment celui décrit dans la demande de brevet EP 0 856 299 qui nécessite l'utilisation d'enveloppe métalliques comportant l'empreinte des prothèses à réaliser. En effet dans la pratique, les essais ont montré que des enveloppes en acier doux permettraient un frittage jusqu'à 1050°C en raison d'un eutectique Ti-Fe à 1085° C, température ne conduisant pas nécessairement à une liaison satisfaisante des microsphères entre elles. Au delà de cette température, il est nécessaire d'utiliser une enveloppe en titane qui, après frittage, risque de poser des problèmes de séparation entre la pièce frittée et son enveloppe. La présente invention au delà des préjugés conduisant à solutionner le problème en abaissant la température de frittage, apporte une solution innovante en s'affranchissant du moule par une consolidation préalable de l'implant permettant au contraire d'augmenter la température de la pièce afin de réunir les conditions nécessaires à un frittage solide.

Parmi les autres préjugés existants, il était également à craindre que les âmes métalliques complexifient excessivement l'opération de moulage, soit en créant des soucis d'étanchéité, soit en créant des problèmes de calage.

Tel n'est cependant pas le cas. En effet il est tout à fait acceptable en pratique de caler la ou les âmes métalliques dans le moule par simple tassement des microsphères autour desdites âmes.

Ainsi l'invention propose comme mode de réalisation spécifique un procédé comprenant une étape supplémentaire (ii') entre les étapes (ii) et (iii) qui consiste à caler ladite au moins une âme massive (7) dans le moule (1) par un élément de maintient.

Par « élément de maintient » on entend tout élément de maintient biocompatible connu de l'homme du métier notamment le titane ou le corail.

Selon un mode de réalisation spécifique de l'invention ledit élément de maintient consiste en au moins un logement pratiqué dans au moins une des parois du moule (1), logement dans lequel au moins l'une des extrémités de ladite au moins une âme massive (7) vient s'insérer.

Selon un autre mode de réalisation spécifique de l'invention ledit élément de maintient consiste en de la poudre de microsphères.

Toute variation ou réalisation présentant les caractéristiques de l'invention devra être considérée comme une forme équivalente et donc comprise dans l'étendue de la protection conférée par la présente demande de brevet.

Selon une certaine forme de réalisation le procédé mis en oeuvre permet d'obtenir avec utilisation d'une densité surfacique d'énergie J prédéterminée comprise entre 5 et 7 J/mm2, préférentiellement 6 J/mm2, un implant dentaire composé d'une part, d'une âme en métal massif (7) comprenant un corps présentant au moins un filetage extérieur (9), éventuellement de pas variable et une tête, évidée en son centre, présentant au moins un moyen permettant d'ancrer une dent, tel qu'un taraudage (11), éventuellement de pas variable et d'autre part d'une superstructure de microsphères (3) formant une collerette placée en périphérie de la tête.

Selon une autre forme de réalisation de l'invention le procédé permet d'obtenir implant destiné à remplacer une mandibule, composé d'une part d'au moins une âme en métal massif (13, 13') s'étendant dans le sens de la longueur de l'implant et comportant au moins un moyen de fixation, tel qu'un trou (17) ou un taraudage et d'autre part d'une superstructure de microsphères formant le corps de la prothèse (15, 15').

Selon une variante de ce mode de réalisation de l'invention l'implant obtenu comporte au moins une extrémité d'au moins une âme en métal massif fait saillie au delà de la superstructure de microsphères de manière à faciliter son ancrage par le chirurgien.

Selon une autre variante de ce mode de réalisation de l'invention l'implant obtenu présente au moins une âme en métal massif située à la périphérie de son bord supérieur et comportant des logements de forme appropriée, tels des taraudages, pour recevoir une implantation dentaire.

Selon une autre variante de l'invention le procédé permet d'obtenir un implant destiné à la thyroplastie et comportant une âme en métal massif formant socle (18) et placée sur celle-ci, une superstructure formée de microsphères (19), sensiblement en forme d'aileron de requin.

Selon une autre variante de l'invention le procédé permet d'obtenir un implant destiné à remplacer une trachée, obtenu selon la revendication 12, caractérisé en ce qu'il est composé d'une part d'au moins une âme en métal massif (19', 19") en forme de bague et comportant au moins un moyen de fixation, tel qu'un trou (17') ou un taraudage et d'autre part d'une superstructure de microsphères formant le corps de la prothèse (20').

Ces éléments seront décrits en détail plus bas.

Il s'agit, bien entendu, d'exemples non limitatifs, l'invention permettant des formes de réalisation extrêmement variées.

Toute variation ou réalisation présentant les caractéristiques de l'invention devra être considérée comme une forme équivalente et donc comprise dans l'étendue de la protection conférée par la présente demande de brevet

Comme abordé plus haut, l'objet de la présente invention se distingue également de l'art antérieur en ce sens qu'il réalise un implant véritablement composite.

Un tel composite est extrêmement innovant, comme nous l'avons vu ci-dessus, car il va à l'encontre même des efforts développés à ce jour et parce que sont hétérogénéité pouvait faire craindre qu'il cumulé les désavantages liés aux propriétés des deux composés sans en avoir les avantages. Ainsi il pouvait être craint que la fragilité inhérente aux métaux à composition ouverte ne fragilise l'ensemble de la structure, il n'est cependant rien. L'âme en métal massif lorsqu'elle est correctement disposée, de taille et de section appropriée se comporte comme une véritable armature augmentant considérablement les propriétés mécaniques de l'ensemble de l'implant sans pour autant diminuer ses propriétés biodynamiques.

L'invention sera mieux comprise, et ses avantages ressortiront plus clairement, au cours de la description suivante des exemples de réalisation, en référence aux dessins annexés dans lesquels :
la figure 1 représente un implant de trachée sans âme massive tel qu'il est connu de l'art antérieur,
la figure 2 représente, en implant dentaire tel qu'il est connu de l'art antérieur, à savoir une vis en titane massif recouvert en partie d'un film bio-actif,
la figure 3 représente, un implant phonatoire réalisé selon l'invention : il est constitué de deux épaulements (4,4') enserrant une pièce cylindrique en titane massif de diamètre 6 mm et de longueur comprise entre 4 et 12 mm selon les caractéristiques anatomiques du patient, cerclée par une zone poreuse composée de microsphères (2) d'épaisseur environ 1 mm.

Lors de la fabrication d'une pièce, l'enchaînement des opérations de préparation et de remplissage du moule est le suivant :
- positionnement de la pièce cylindrique en titane massif dans le moule où les électrodes définissent les limites de la zone poreuse constituée de microsphères autour de l'âme en titane massif,
- fermeture du moule,
- remplissage du volume interne du moule par de la poudre de microsphères (environ 0,5 g pour une longueur de 8 mm),
- serrage de l'ensemble à l'aide des vis de réglage pour empêcher tout mouvement et assurer un parfait contact entre les différents éléments,
- raccordement de chacune des électrodes à une des deux polarités du banc de condensateurs puis soudage par électro-étincelage avec une énergie de 1 200 J soit 6 J/mm2, le courant s'écoulant radialement, vers l'âme de la pièce en titane massif entourée par la zone poreuse constituée des microsphères,
- démontage du moule et récupération de la pièce,

La pièce est ensuite consolidée par frittage à haute température sous vide poussé. Elle est ensuite nettoyée par ultra-sons dans un solvant approprié puis stérilisée.

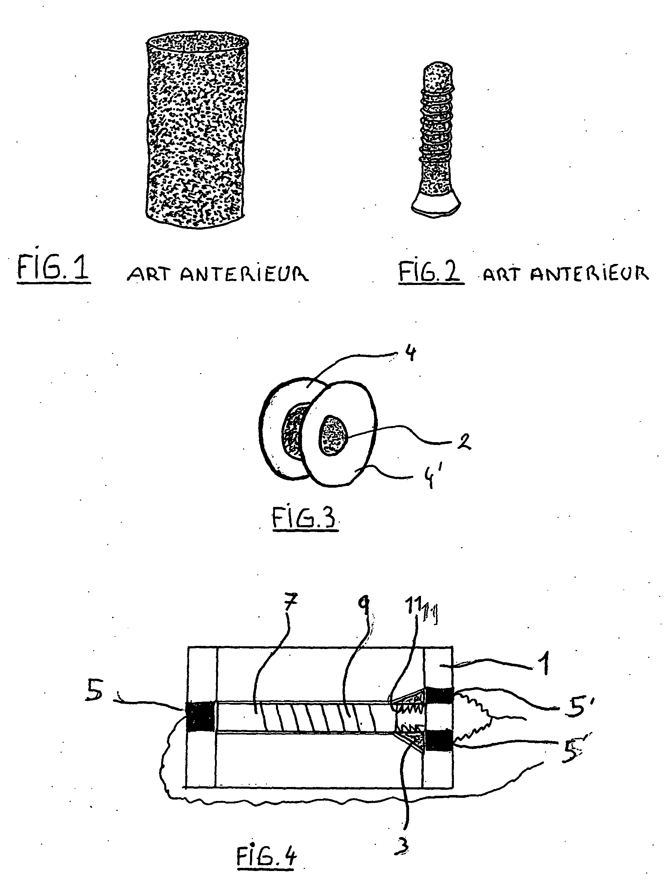

la figure 4 représente, en coupe longitudinale un implant dentaire en forme de vis (7) en position dans son moule (1) réalisé en matière non conductrice, telle de l'altuglas ® une première électrode (5) étant en contact avec l'âme de la pièce en titane massif formant le corps de la vis (7) et une seconde paire d'électrodes (5') de polarité opposée à la première électrode (5) étant en contact avec la partie poreuse de la tête de vis constituée de microsphères (3), l'âme de la pièce (7) formant électrode intermédiaire lors de l'électro-étincelage.

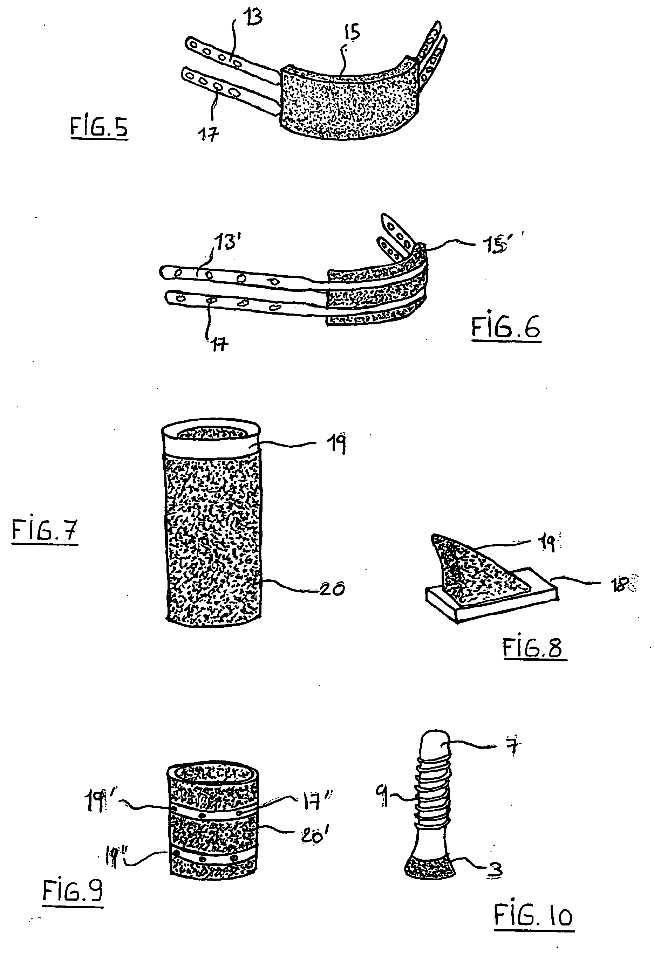

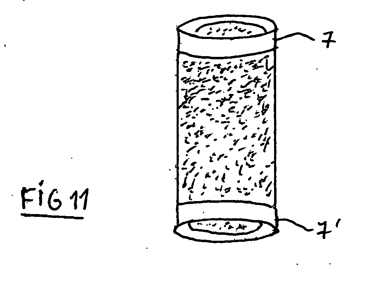

La figure 5 représente, une prothèse mandibulaire munie de deux âmes en forme de bandes de renfort en titane massif (13) qui permettent la fixation de l'implant dans l'os restant de la mandibule, à cette fin des trous (17) sont percés vers leurs extrémités, en dehors de la zone poreuse (15) constituée de microsphères, pour le passage des vis.
Lors de la fabrication d'une pièce, l'enchaînement des opérations de préparation et de remplissage du moule est le suivant :
- sur l'une des moitiés du moule, positionnement des âmes maintenues en place par des vis,
- fermeture du moule, mise en place des deux électrodes en laiton,
- remplissage du volume intérieur du moule où sont situées les deux âmes, par de la poudre de microsphères, par exemple 10 g pour une mandibule d'épaisseur 4 mm, de hauteur 20 mm et de longueur développée totale 45,25 mm,
- serrage de l'ensemble à l'aide des vis de réglage pour empêcher tout mouvement et assurer un parfait contact entre les différents éléments,
- raccordement de chacune des électrodes à une des deux polarités du banc de condensateurs puis soudage par électro-étincelage, le courant de décharge s'écoulant perpendiculairement aux bandes de renfort, l'énergie nécessaire étant de 1 380 J soit 7,6 J/mm2,
- démontage du moule et récupération de la pièce.

La pièce est ensuite consolidée par frittage à haute température sous vide poussé. Elle est ensuite nettoyée par ultra-sons dans un solvant approprié puis stérilisée.

La figure 6 représente une variante de la prothèse décrite en figure 5, où les deux âmes servant de bande de renfort (13') sont de d'une section permettant leur affleurement du côté externe de la partie de la prothèse constituée de microsphères (15'), par exemple l'épaisseur de ces bandes de renfort, usinées dans de la tôle de titane, est de 1 mm et leur largeur de 5 mm ; celle-ci peut être réduite, par exemple à 2,5 mm, dans la zone poreuse pour augmenter le volume colonisable par les tissus.

La figure 7 représente une prothèse de trachée comportant une âme en titane massif formant bague de renforcement (19) et en corps cylindrique (20) formé de microsphères, l'enchaînement des opérations de remplissage du moule non conducteur est le suivant, pour des trachées de diamètre extérieur 25 mm et de diamètre intérieur 22 mm :
- positionnement d'une des deux électrodes en laiton à l'une des extrémités du moule, l'autre extrémité étant maintenue ouverte,
- mise en place au contact de l'électrode de la bague d'extrémité en titane massif (19) formant électrode intermédiaire,
- mise en place d'une certaine quantité de poudre de microsphères, de façon à constituer la hauteur de titane poreux requise (par exemple 1,6 g pour une hauteur de 5 mm) ; nivelage à l'aide d'un outil approprié de la surface de poudre et tassage par vibrations,
- positionnement de la seconde électrode en laiton en contact avec la poudre de microsphères et fermeture du moule,
- serrage de l'ensemble à l'aide des vis de réglage pour empêcher tout mouvement et assurer un parfait contact entre les différents éléments,
- raccordement de chacune des électrodes à une des deux polarités du banc de condensateurs puis soudage par électro-étincelage avec une énergie totale de 900 J soit environ 6 J/mm2, le courant s'écoulant longitudinalement en traversant les différents éléments de l'empilement,
- démontage du moule et récupération de la pièce.

La pièce est ensuite consolidée par frittage à haute température sous vide poussé, puis mise à longueur par découpe à la meule. Elle est ensuite nettoyée par ultra-sons dans un solvant approprié, puis stérilisée.

La figure 8 représente une pièce de thyroplastie munie d'une âme en titane massif formant embase (18), et d'une superstructure composée de microsphères (19).

Lors de la fabrication d'une pièce, l'enchaînement des opérations de préparation et de remplissage du moule est le suivant :
- mise en place de l'embase en titane massif (18) à l'extrémité de l'électrode en laiton en contact avec la base de la pièce
- mise en place des électrodes et fermeture du moule,
- remplissage du volume interne du moule par de la poudre de microsphères (environ 0,5 g),
- serrage de l'ensemble à l'aide des vis de réglage pour empêcher tout mouvement et assurer un parfait contact entre les différents éléments,
- raccordement de chacune des électrodes à une des deux polarités du banc de condensateurs puis soudage par électro-étincelage avec une énergie de 200 J soit environ 4 J/mm2, le courant s'écoulant axialement,
- démontage du moule et récupération de la pièce.

La pièce est ensuite consolidée par frittage à haute température sous vide poussé. Elle est ensuite nettoyée par ultra-sons dans un solvant approprié puis stérilisée.

La figure 9 représente une prothèse de trachée comportant deux âmes en titane massif formant bagues de renforcement (19' et (19") et en corps cylindrique (20') formé de microsphères, l'enchaînement des opérations de remplissage du moule est le suivant, pour des trachées de diamètre extérieur 25 mm et de diamètre intérieur 22 mm (épaisseur de paroi : 1,5 mm) :
- positionnement d'une des deux électrodes en laiton à l'une des extrémités du moule non conducteur, l'autre extrémité étant maintenue ouverte,
- mise en place, au contact de l'électrode, d'une certaine quantité de poudre, de façon à constituer la hauteur de titane poreux requise (par exemple 3,2 g pour une hauteur de 10 mm) ; nivelage à l'aide d'un outil approprié de la surface de poudre et tassage par vibrations,
- mise en place au dessus de la poudre d'une première bague en titane massif (19') formant électrode intermédiaire,
- mise place d'une quantité de poudre permettant d'obtenir la hauteur de titane poreux appropriée (par exemple 3,8 g pour une hauteur de 12 mm) ; nivelage à l'aide d'un outil approprié de la surface de poudre et tassage par vibrations,
- mise en place au dessus de la poudre de la deuxième bague en titane massif (19") formant électrode intermédiaire,
- mise en place d'une certaine quantité de poudre, de façon à constituer la hauteur de titane poreux requise (par exemple 3,2 g pour une hauteur de 10 mm) ; nivelage à l'aide d'un outil approprié de la surface de poudre et tassage par vibrations,
- positionnement de la seconde électrode en laiton au contact de la poudre de microsphères et fermeture du moule,
- serrage de l'ensemble à l'aide des vis de réglage pour empêcher tout mouvement et assurer un parfait contact entre les différents éléments,
- raccordement de chacune des électrodes à une des deux polarités du banc de condensateurs puis soudage par électro-étincelage avec une énergie totale de 900 J soit environ 6 J/mm2,
- démontage du moule et récupération de la pièce.

La pièce est ensuite consolidée par frittage à haute température sous vide poussé, puis mise à longueur par découpe à la meule. Elle est ensuite nettoyée par ultra-sons dans un solvant approprié puis stérilisée.

La figure 10 montre un implant dentaire formé d'une âme en titane massif (7) de forme cylindrique muni à l'extérieur, à une extrémité, d'un filetage (9) destiné à être vissé dans l'os de la mandibule et à l'intérieur d'un taraudage destiné à permettre la fixation de la dent de remplacement.

La zone en titane poreux formée de microsphères (3) sera située à la partie supérieure, au dessus de l'os, en contact avec les tissus de la gencive.

Lors de la fabrication d'une pièce, l'enchaînement des opérations de préparation et de remplissage du moule est le suivant :
- positionnement de l'implant en titane massif (7) formant électrode intermédiaire dans le moule non-conducteur et verrouillage de son positionnement,
- mise en place des électrodes suivant la description faite en figure 4 et verrouillage de leur position,
- remplissage du volume interne du moule par de la poudre de microsphères (environ 0,1 g),
- serrage de l'ensemble à l'aide des vis de réglage pour empêcher tout mouvement et assurer un parfait contact entre les différents éléments,
- raccordement de chacune des électrodes à une des deux polarités du banc de condensateurs puis soudage par électro-étincelage avec une énergie d'environ 50 J soit 6 J/mm2, le courant s'écoulant longitudinalement dans l'implant en titane massif,
- démontage du moule et récupération de la pièce.

La liaison des microsphères entre elles et à la partie massive est ensuite consolidée par frittage à haute température sous vide poussé. La pièce est ensuite nettoyée par ultra-sons dans un solvant approprié puis stérilisée.

Bien évidemment, tout équivalent au titane massif et au titane microporeux, c'est-à-dire présentant des caractéristiques similaires pourraient être envisagé et donc devrait être considéré comme moyen équivalent.

Bien évidemment, les différentes parties des exemples ci-dessus selon l'invention sont dimensionnées de manière à pouvoir correspondre à la morphologie du patient et présenter les meilleurs qualités recherchées selon l'invention à savoir des qualité mécaniques et bioactives.

## Revendications

1. Implant métallique composite de soutien et/ou de remplacement tissulaire comprenant au moins une âme en métal massif et au moins une superstructure métallique à porosité ouverte et homogène constituée de microsphères métalliques obtenu par un procédé comprenant les étapes suivantes .
- (i) sélectionner un moule (1), non-conducteur, de forme appropriée correspondant à l'implant souhaité,
- (ii) disposer dans ledit moule au moins une âme massive métallique (7),
- (iii) remplir le volume du moule (1) restant disponible par de la poudre de microsphères (3), et
(iv) solidariser les microsphères (3) entre elles ainsi qu'à ladite au moins une âme massive (7) par électro-étincelage, ladite étape iv) d'électro-étincelage étant réalisée par décharge d'un courant électrique, d'une tension prédéterminée de manière à obtenir une densité surfacique d'énergie J inférieur ou égale à 10 J/mm² entre, d'une part, au moins une première électrode (5) en périphérie du moule (1) et, d'autre part, au moins une seconde électrode (5') de polarité opposée et positionnée de manière à ce que les lignes de courant formées entre ces deux électrodes ne soient pas parallèles à l'interface microsphères/âme métalliques (7) de plus grande surface ;
**caractérisé en ce que** la ou les âmes en métal massif servent d'électrode lors de la fabrication dudit implant, et que la superstructure métallique à porosité ouverte et homogène est obtenue par solidarisation de microsphères suite au passage d'un courant électrique dans lesdites électrodes.

2. Implant métallique composite de soutien et/ou de remplacement tissulaire selon la revendication 1, **caractérisé en ce que** la ou les âmes en métal massif ainsi que les microsphères sont indépendamment constituées d'un métal sélectionné parmi le titane, les alliages de titane ou les alliages biocompatibles.

3. Implant métallique de soutien et/ou de remplacement tissulaire selon l'une quelconque des revendications 1 à 2, **caractérisé en ce qu'**il est composé d'une part d'au moins une âme en métal massif (19' et 19") en forme de bague et comportant au moins un moyen de fixation, tel qu'un trou (17') ou un taraudage, et d'autre part d'une superstructure de microsphères formant le corps de la prothèse (20').

4. Implant métallique de soutien et/ou de remplacement tissulaire selon la revendication 3, **caractérisé en ce que** ledit implant est un implant destiné à remplacer une trachée.

5. Implant métallique de soutien et/ou de remplacement tissulaire selon l'une quelconque des revendications 1 à 2, **caractérisé en ce qu'**il est composé d'une part, d'une âme en métal massif (7) comprenant un corps présentant au moins un filetage extérieur (9), éventuellement de pas variable et une tête, évidée en son centre, présentant au moins un moyen permettant d'ancrer une dent, tel qu'un taraudage (11) éventuellement de pas variable, et d'autre part d'une superstructure de microsphères (3) formant une collerette placée en périphérie de la tête.

6. Implant métallique de soutien et/ou de remplacement tissulaire selon la revendication 5, **caractérisé en ce que** ledit implant est un implant dentaire.

7. Implant métallique de soutien et/ou de remplacement tissulaire selon l'une quelconque des revendications 1 à 2, **caractérisé en ce qu'**il est composé d'une âme en métal massif formant embase (18) et placée sur celle-ci, d'une superstructure métallique composée de microsphères (21) sensiblement en forme d'aileron de requin.

8. Implant métallique de soutien et/ou de remplacement tissulaire selon la revendication 7, **caractérisé en ce que** ledit implant est un implant destiné à la thyroplastie.

9. Implant métallique de soutien et/ou de remplacement tissulaire selon quelconque des revendication 1 à 2, **caractérisé en ce qu'**il est composé d'une part d'au moins une âme en métal massif (13, 13') s'étendant dans le sens de la longueur de l'implant et comportant au moins un moyen de fixation, tel qu'un trou (17) ou un taraudage et d'autre part d'une superstructure de microsphères formant le corps de la prothèse (15, 15').

10. Implant selon la revendication 9, **caractérisé en ce qu'**au moins une extrémité d'au moins une âme en métal massif fait saillie (13, 13') de la superstructure de microsphères (15, 15') de manière à faciliter son ancrage par le chirurgien.

11. Implant métallique de soutien et/ou de remplacement tissulaire selon l'une quelconque des revendication 9 ou 10 **caractérisé en ce que** ledit implant est un implant destiné à remplacer une mandibule.

## Claims

1. Composite metal implant for supporting and/or replacing tissue, comprising at least one solid metal core and at least one metal superstructure, of open and homogeneous porosity, formed by metal microspheres, said implant being obtained by a method comprising the following steps:
- (i) selecting a non-conducting mould (1) of a suitable shape which corresponds to the desired implant,
- (ii) arranging at least one solid metal core (7) in said mould,
- (iii) filling the volume of the mould (1) that remains available with microsphere powder (3), and
- (iv) connecting the microspheres (3) to each other as well as to said at least one solid core (7) by electrical discharge machining, said electrical discharge machining step iv) being carried out by discharging an electric current of a predetermined voltage so as to obtain a surface energy density J that is less than or equal to 10 J/mm² between, on one hand, at least a first electrode (5) at the periphery of the mould (1) and, on the other hand, at least a second electrode (5') of opposite polarity and positioned in such a way that the current lines formed between these two electrodes are not parallel to the interface between the microspheres and metal core (7) of greater surface area;
**characterised in that** the solid metal core(s) act(s) as an electrode during the production of said implant, and **in that** the metal superstructure of open and homogeneous porosity, is obtained by connecting microspheres by passing an electric current through said electrodes.

2. Composite metal implant for supporting and/or replacing tissue according to claim 1, **characterised in that** the solid metal core(s) and the microspheres are formed independently from a metal chosen from among titanium, titanium alloys or bio-compatible alloys.

3. Metal implant for supporting and/or replacing tissue according to either claim 1 or claim 2, **characterised in that** it is made up, on the one hand, of at least one solid metal core (19' and 19") of annular shape and comprising at least one fixing means, such as a hole (17') or an internal thread, and, on the other hand, of a superstructure of microspheres forming the body of the prosthesis (20').

4. Metal implant for supporting and/or replacing tissue according to claim 3, **characterised in that** said implant is an implant intended to replace a trachea.

5. Metal implant for supporting and/or replacing tissue according to either claim 1 or claim 2, **characterised in that** it is made up, on the one hand, of a solid metal core (7) comprising a body having at least an external thread (9), optionally of variable pitch, and a head, hollowed out in its centre, having at least a means for anchoring a tooth, such as an internal thread (11), optionally of variable pitch, and, on the other hand, of a superstructure of microspheres (3) forming a flange positioned at the periphery of the head.

6. Metal implant for supporting and/or replacing tissue according to claim 5, **characterised in that** said implant is a dental implant.

7. Metal implant for supporting and/or replacing tissue according to either claim 1 or claim 2, **characterised in that** it is made up of a solid metal core which forms a base (18) and, positioned thereon, of a metal superstructure made up of microspheres (21), substantially in the shape of a shark fin.

8. Metal implant for supporting and/or replacing tissue according to claim 7, **characterised in that** said implant is an implant intended for thyroplasty.

9. Metal implant for supporting and/or replacing tissue according to either claim 1 or claim 2, **characterised in that** it is made up, on the one hand, of at least one solid metal core (13, 13') extending in the direction of the length of the implant and comprising at least one fixing means, such as a hole (17) or an internal thread, and, on the other hand, of a superstructure of microspheres forming the body of the prosthesis (15, 15').

10. Implant according to claim 9, **characterised in that** at least one end of at least one solid metal core (13, 13') projects from the superstructure of microspheres (15, 15') so as to facilitate its anchoring by the surgeon.

11. Metal implant for supporting and/or replacing tissue according to either claim 9 or claim 10, **characterised in that** said implant is an implant intended to replace a mandible.

## Patentansprüche

1. Komposit-Metallimplantat zum Unterstützen und/oder zum Ersetzen von Gewebe, das mindestens einen Kern aus massivem Metall und mindestens eine metallische Überstruktur mit offener und homogener Porosität umfasst, die aus metallischen Mikrokügelchen gebildet ist, das mit einem Verfahren hergestellt ist, das die folgenden Schritte umfasst:
- (i) Wählen einer nicht leitenden Form (1) mit einer geeigneten Form, die dem gewünschten Implantat entspricht,
- (ii) Anordnen von mindestens einem massiven metallischen Kern (7) in der Form,
- (iii) Auffüllen des verfügbar bleibenden Volumens der Form (1) mit Pulver aus Mikrokügelchen (3), und
- (iv) Verbinden der Mikrokügelchen (3) untereinander sowie mit dem mindestens einen massiven Kern (7) durch Elektroerodieren, wobei der Schritt iv) des Elektroerodierens durch Entladung eines elektrischen Stroms einer festgelegten Spannung derart erfolgt, dass eine Energieflächendichte J von unter oder gleich 10 J/mm² erhalten wird, zwischen einerseits mindestens einer ersten Elektrode (5) am Rand der Form (1) und andererseits mindestens einer zweiten Elektrode (5') entgegengesetzter Polarität, die derart angeordnet ist, dass die Stromlinien, die sich zwischen diesen beiden Elektroden bilden, nicht parallel zur Grenzfläche metallische Mikrokügelchen/metallischer Kern (7) mit der größeren Fläche verlaufen;
**dadurch gekennzeichnet, dass** der Kern oder die Kerne aus massivem Metall bei der Herstellung des Implantats als Elektrode dient bzw. dienen und dass die metallische Überstruktur mit offener und homogener Porosität durch Verbinden von Mikrokügelchen nach Durchgang eines elektrischen Stroms in den Elektroden erhalten wird.

2. Komposit-Metallimplantat zum Unterstützen und/oder zum Ersetzen von Gewebe nach Anspruch 1, **dadurch gekennzeichnet, dass** der Kern oder die Kerne aus massivem Metall sowie die Mikrokügelchen unabhängig aus einem Metall ausgewählt aus Titan, Titanlegierungen oder biokompatiblen Legierungen gebildet sind.

3. Metallimplantat zum Unterstützen und/oder zum Ersetzen von Gewebe nach einem der Ansprüche 1 bis 2, **dadurch gekennzeichnet, dass** es einerseits aus mindestens einem Kern aus massivem Metall (19' und 19") in Form eines Rings, der mindestens ein Befestigungsmittel wie eine Bohrung (17') oder ein Innengewinde aufweist, und andererseits aus einer Überstruktur aus Mikrokügelchen besteht, die den Körper der Prothese (20') bildet.

4. Metallimplantat zum Unterstützen und/oder zum Ersetzen von Gewebe nach Anspruch 3, **dadurch gekennzeichnet, dass** das Implantat ein Implantat ist, das eine Luftröhre ersetzen soll.

5. Metallimplantat zum Unterstützen und/oder zum Ersetzen von Gewebe nach einem der Ansprüche 1 bis 2, **dadurch gekennzeichnet, dass** es einerseits aus einem Kern aus massivem Metall (7), der einen Körper, der mindestens ein Außengewinde (9), gegebenenfalls mit variabler Steigung, aufweist, und einen Kopf umfasst, der in der Mitte hohl ist und mindestens ein Mittel, mit dem ein Zahn verankert werden kann, beispielsweise ein Innengewinde (11), gegebenenfalls mit variabler Steigung, aufweist, und andererseits aus einer Überstruktur aus Mikrokügelchen (3) besteht, die einen Bund bildet, der am Rand des Kopfs platziert ist.

6. Metallimplantat zum Unterstützen und/oder zum Ersetzen von Gewebe nach Anspruch 5, **dadurch gekennzeichnet, dass** das Implantat ein Zahnimplantat ist.

7. Metallimplantat zum Unterstützen und/oder zum Ersetzen von Gewebe nach einem der Ansprüche 1 bis 2, **dadurch gekennzeichnet, dass** es aus einem Kern aus massivem Metall, der eine Befestigungsfläche (18) bildet, und darauf platziert aus einer metallischen Überstruktur besteht, bestehend aus Mikrokügelchen (21), im Wesentlichen in Form einer Haifischflosse.

8. Metallimplantat zum Unterstützen und/oder zum Ersetzen von Gewebe nach Anspruch 7, **dadurch gekennzeichnet, dass** das Implantat ein Implantat ist, das für die Thyroplastik bestimmt ist.

9. Metallimplantat zum Unterstützen und/oder zum Ersetzen von Gewebe nach einem der Ansprüche 1 bis 2, **dadurch gekennzeichnet, dass** es einerseits aus mindestens einem Kern aus massivem Metall (13, 13'), der sich in Richtung der Länge des Implantats erstreckt und mindestens ein Befestigungsmittel wie eine Bohrung (17) oder ein Innengewinde aufweist, und andererseits aus einer Überstruktur aus Mikrokügelchen besteht, die den Körper der Prothese (15, 15') bildet.

10. Implantat nach Anspruch 9, **dadurch gekennzeichnet, dass** mindestens ein Ende von mindestens einem Kern aus massivem Metall (13, 13') aus der Überstruktur aus Mikrokügelchen (15, 15') ragt, um vom Chirurgen einfacher verankert werden zu können.

11. Metallimplantat zum Unterstützen und/oder zum Ersetzen von Gewebe nach einem der Ansprüche 9 oder 10, **dadurch gekennzeichnet, dass** das Implantat ein Implantat ist, das einen Unterkiefer ersetzen soll.
